(19) Europäisches Patentamt
European Patent Office
Office européen des brevets

(11) Numéro de publication : **0 406 065 B1**

(12) # FASCICULE DE BREVET EUROPEEN

(45) Date de publication du fascicule du brevet : **07.04.93 Bulletin 93/14**

(51) Int. Cl.$^5$ : **C07C 49/203,** C07C 45/67, C07C 69/738, C07C 403/00

(21) Numéro de dépôt : **90401738.1**

(22) Date de dépôt : **20.06.90**

(54) **Procédé de préparation de la pseudo-ionone.**

(30) Priorité : **22.06.89 FR 8908317**

(43) Date de publication de la demande : **02.01.91 Bulletin 91/01**

(45) Mention de la délivrance du brevet : **07.04.93 Bulletin 93/14**

(84) Etats contractants désignés : **AT BE CH DE DK ES FR GB GR IT LI LU NL SE**

(56) Documents cités :
**EP-A- 0 082 781**
**FR-A- 2 526 420**
**JOURNAL OF SYNTHETIC ORGANIC CHEMISTRY, no. 11, 1978, pages 992-998; I. MINAMI et a.: "New Methods for the Syntheses of alpha,beta-Unsaturated Ketones, Aldehydes, and Nitriles by the Palladium-Catalyzed Reactions of Allyl beta-Oxo Esters, Allyl 1-Alkenyl Carbonates, and Allyl alpha-Cyano Esters"**

(56) Documents cités :
**JOURNAL OF THE AMERICAN CHEMICAL SOCIETY, vol. 104, no. 21, 20 octobre 1982, pages 5844-5846; I. SHIMIZU et al: "Palladium-Catalyzed Decarboxylation-Dehydrogenation of Allyl beta-Keto Carboxylates and Allyl Enol Carbonates as a Novel Synthetic Method for alpha-Substituted alpha,beta-Unsaturated Ketones"**

(73) Titulaire : **RHONE-POULENC SANTE**
**20, avenue Raymond Aron**
**F-92160 Antony (FR)**

(72) Inventeur : **Mercier, Claude**
**85 Avenue du Point du Jour**
**F-69005 Lyon (FR)**
Inventeur : **Mignani, Gérard**
**2 Avenue des Frères Lumière**
**F-69008 Lyon (FR)**

(74) Mandataire : **Le Pennec, Magali et al**
**RHONE-POULENC SANTE Service Brevets Santé, 20 Avenue Raymond Aron**
**F-92160 Antony (FR)**

**EP 0 406 065 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de préparation de la pseudo-ionone qui est un intermédiaire particulièrement intéressant pour réaliser la synthèse de la vitamine A.

D'après le brevet français FR 81 23684 (2 518 538), il est connu de préparer la pseudo-ionone en traitant un β-cétoester chloré de formule générale :

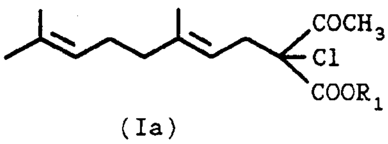

(Ia)

ou

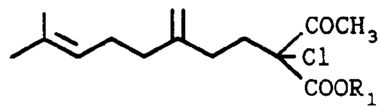

(Ib)

dans laquelle $R_1$ représente un radical alkyle contenant 1 à 4 atomes de carbone par un système chlorure de lithium-acide minéral-amine tertiaire dans un solvant aprotique polaire basique à une température comprise entre 80 et 160°C. Les produits de formule générale (Ia) et (Ib) peuvent être obtenus par action du chlorure cuivrique en présence de chlorure de lithium dans un solvant aprotique basique sur un produit de formule générale :

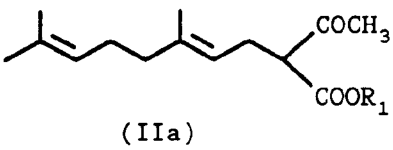

(IIa)

ou

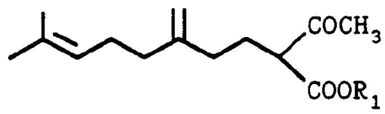

(IIb)

qui est lui-même obtenu par action d'un acétylacétate d'alkyle sur le myrcène dans les conditions décrites dans le brevet européen EP 44771.

I. Minami et coll., Synthesis, p. 992 (1987) et le brevet français FR 83 07418 (2 526 420) enseignent que la décarboxylation oxydante ne peut être réalisée que sur des β-cétoesters allyliques α,α-disubstitués.

Il a maintenant été trouvé, et c'est ce qui fait l'objet de la présente invention, que la pseudo-ionone peut être obtenue par décarboxylation oxydante des β-cétoesters allyliques monosubstitués en α de formule :

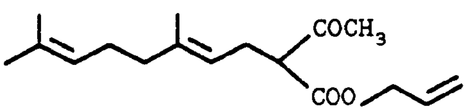

(IIIa)

ou

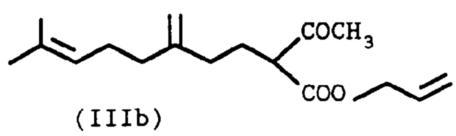

(IIIb)

en opérant, à une température comprise entre 0°C et la température de reflux du mélange réactionnel, dans un solvant organique choisi parmi les amides (diméthylformamide), les nitriles (acétonitrile) et les dinitriles (méthylglutaronitrile) adiponitrile, dicyano-1,6 hexane) en présence d'un catalyseur à base de palladium tel l'acétate de palladium [Pd(OAc)$_2$] ou le bis(dibenzylidène acétone) palladium [Pd(dba)$_2$] et éventuellement d'un ligand tel qu'une phosphine comme la triphénylphosphine ou le diphénylphosphinoéthane.

Le procédé selon l'invention peut être mis en oeuvre soit sur les produits de formule (IIIa) ou (IIIb) préalablement isolés soit sur le mélange des produits de formules (IIIa) et (IIIb) conduisant ainsi, selon le cas aux produits de formule :

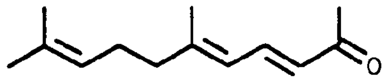

(IVa ; pseudo-ionone)

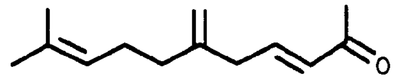

(IVb)

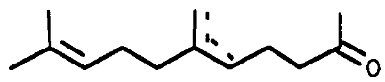

(IVc)

éventuellement en mélange.

Le produit de formule (IVb) peut être facilement isomérisé en pseudo-ionone de formule (IVa) selon les techniques décrites par Y. Fujita et coll., Tetrahedron Letters, 1347 (1980), cette isomérisation pouvant être réalisée soit sur le produit (IVb) isolé soit sur le produit (IVb) en mélange avec la pseudo-ionone (IVa).

Afin d'augmenter la sélectivité, c'est-à-dire afin d'éviter la formation du produit de formule (IVc), et d'accroître la réactivité, il est particulièrement avantageux de mettre en oeuvre le procédé en utilisant un dinitrile comme solvant. Dans ces conditions, il est possible de mettre en oeuvre le procédé à une température comprise de préférence entre 0 et 30°C.

Généralement, le procédé selon l'invention est mis en oeuvre sur un mélange des produits de formule (IIIa) et (IIIb) qui peut être obtenu par transestérification catalysée par le sodium des β-cétoesters méthyliques obtenus par condensation du myrcène sur l'acétylacétate d'éthyle dans les conditions décrites dans le brevet européen EP 44 771.

Le produit de formule (IIIa) peut aussi être obtenu par condensation du chlorure de géranyle sur l'acétoacétate d'allyle préalablement anionisé au moyen par exemple de sodium métallique ou d'un hydrure de métal alcalin tel que l'hydrure de sodium, en opérant dans un solvant organique anhydre choisi parmi les éthers comme le tétrahydrofuranne.

L'acétoacétate d'allyle peut être obtenu selon le procédé décrit par W. Kimel et coll., J. Amer. Chem. Soc., 65, 1992 (1943).

Les produits de formule (IIIa) et (IIIb) sont des produits nouveaux qui constituent un autre objet de la présente invention.

Les exemples suivants, donnés à titre non limitatif, montrent comment l'invention peut être mise en pratique.

EXEMPLE 1

Dans un ballon tricol de 50 cm3, on introduit, sous atmosphère d'argon, 2,78 g d'acétyl-2 diméthyl-5,9 décadiène-4,8 oate d'allyle (10 m.moles), 45 mg d'acétate de palladium et 10 cm3 d'acétonitrile. On chauffe au reflux pendant 1 heure 30 minutes. Après refroidissement, le mélange réactionnel est extrait au pentane. Après évaporation du solvant, on obtient 1,78 g d'un produit brut contenant la pseudo-ionone et la géranylacétone dans le rapport molaire 91/9.

La structure des produits obtenus est confirmée par l'analyse par chromatographie en phase vapeur et par les spectres infra-rouge, de masse et de résonance magnétique nucléaire du proton.

Le rendement est de 93 %.

L'acétyl-2 diméthyl-5,9 décadiène-4,8 oate d'allyle utilisé comme produit de départ peut être préparé de la manière suivante :

Dans un ballon tricol de 250 cm3, on introduit, sous atmosphère d'argon, 15,4 g d'acétoacétate d'allyle (0,1 mole), 70 cm3 de tétrahydrofuranne et 2,3 g de sodium métallique. On laisse en contact pendant 2 heures puis on ajoute 17,5 g de chlorure de géranyle (0,1 mole). On chauffe à reflux pendant 16 heures. Après refroidissement, le mélange réactionnel est versé dans 400 cm3 de saumure saturée puis on extrait au pentane. Après séchage de la phase organique sur sulfate de sodium, filtration et évaporation du solvant, on obtient 26,1 g d'un produit brut qui par distillation à 128-131°C sous pression réduite (0,3 mm de mercure ; 0,04 kPa) fournit 19,5 g d'acéthyl-2 diméthyl-5,9 décadiène-4,8 oate d'allyle dont la pureté est supérieure à 90 %.

Le rendement est voisin de 70 %.

La structure du produit obtenu est confirmée par les spectres infra-rouge, de masse et de résonance magnétique nucléaire du proton.

EXEMPLE 2

Dans un ballon tricol de 50 cm3, on introduit, sous atmosphère d'argon, 2,78 g d'un mélange 55/45 de β-cétoesters allyliques représentés par les formules (IIIa) et (IIIb) (10 m.moles), 24 mg d'acétate de palladium et 10 $cm^3$ d'adiponitrile. On agite le mélange réactionnel pendant 1 heure à 20°C.

L'analyse par chromatographie en phase vapeur du produit brut de réaction montre que le taux de transformation est de 100 % et que le rendement de la réaction est de 91 %.

Le produit obtenu est constitué de :

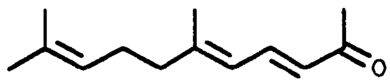

(E,E) : 46 % (IVa)

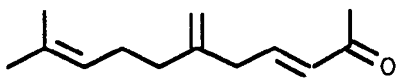

: 44 % (IVb)

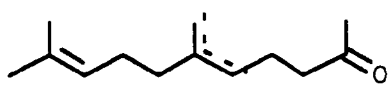

: 8 % (IVc)

La structure des produits obtenus est confirmée par le spectre de masse, le spectre infra-rouge et le spectre de résonance magnétique nucléaire du proton étudiés en couplage avec la chromatographie en phase vapeur.

Le mélange (55/45) des β-cétoesters allyliques de formule (IIIa) et (IIIb) peut être obtenu de la manière suivante :

Dans un ballon tricol de 250 cm3, on introduit, sous atmosphère d'argon, 84 g d'alcool allylique (1,45 mole) puis on ajoute 0,34 g de sodium métallique. Lorsque le sodium est totalement dissous, on ajoute 70 g du mélange de β-cétoesters méthyliques (0,28 mole) puis on chauffe le mélange réactionnel pendant 6 heures 15 minutes à 90°C. Par distillation, on élimine l'alcool allylique en excès puis on isole 54 g du mélange 55/45 des β-cétoesters allyliques ($P.E._{0,013\ kPa}$ = 121°C), dont la pureté est voisine de 95 %.

La structure du produit obtenu est confirmée par le spectre infra-rouge, le spectre de masse et le spectre de résonance magnétique nucléaire du proton.

EXEMPLES 3 A 7

On opère comme dans l'exemple 2 dans les conditions précisées dans le tableau I.

TABLEAU I

| Exemple | Solvant | Catalyseur | Conditions | Résultats TT % | Rdt % | IVa % | IVb % | IVc % |
|---|---|---|---|---|---|---|---|---|
| 3 | Méthylglutaronitrile | $Pd(oAc)_2$ 1 % | 20°C 2 heures | 95 | 92 | 42 | 45 | 13 |
| 4 | Dicyano-1,6 hexane | $Pd(oAc)_2$ 1 % | 20°C 2 heures | 98 | 90 | 43 | 48 | 9 |
| 5 | Acétonitrile | $Pd(oAc)_2$ 3,4 % | 90°C 1 heure | 100 | 88 | 46 | 32 | 22 |
| 6 | Benzonitrile | $Pd(oAc)_2$ 1 % | 80°C 1 heure | 100 | 89 | 48 | 40 | 12 |
| 7 | Benzonitrile | $Pd(dba)_2$ | 80°C 1 heure | 100 | 93 | 52 | 39 | 9 |

## Revendications

1. Procédé de préparation des produits de formule :

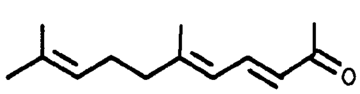

ou

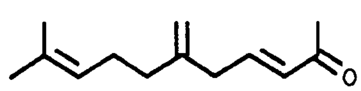

pris seuls ou en mélange, caractérisé en ce que l'on effectue la décarboxylation oxydante d'un β-cétoester

allylique de formule :

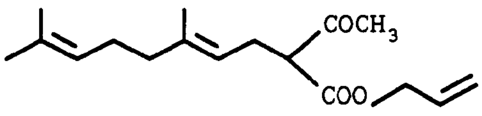 ou 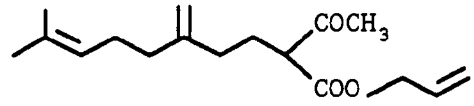

pris seul ou en mélange en opérant dans un solvant organique choisi parmi les amides, les nitriles et les dinitriles en présence d'un catalyseur à base de palladium et éventuellement d'un ligand.

**2.** Procédé selon la revendication 1 caractérisé en ce que le solvant est choisi parmi le diméthylformamide, l'acétonitrile, le benzonitrile, le méthylglutaronitrile, l'adiponitrile et le dicyano-1,6 hexane.

**3.** Procédé selon la revendication 1 caractérisé en ce que le catalyseur est choisi parmi $[Pd(OAc)_2]$ et $[Pd(dba)_2]$.

**4.** Procédé selon la revendication 1 caractérisé en ce que le ligand est choisi parmi la triphénylphosphine et le diphénylphosphinoéthane.

**5.** Procédé selon la revendication 1 caractérisé en ce que l'on opère à une température comprise entre 0°C et la température de reflux du mélange réactionnel.

**6.** Les produits de formule :

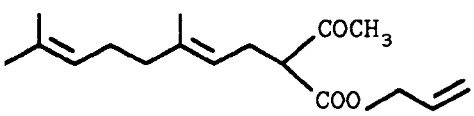 et 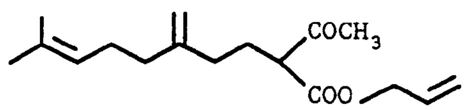

**Patentansprüche**

**1.** Verfahren zur Herstellung der Produkte der Formel

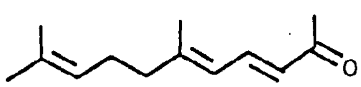 oder 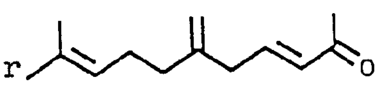

allein oder im Gemisch, dadurch gekennzeichnet, daß man eine oxidierende Decarboxylierung eines allylischen β-Ketoesters der Formel

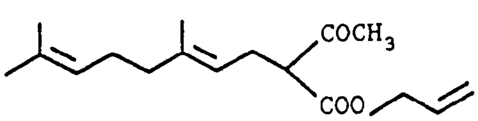 oder 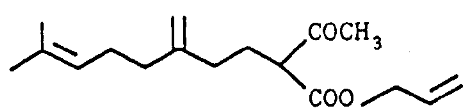

allein oder im Gemisch vornimmt, wobei man in einem organischen Lösungsmiitel, ausgewählt unter den Amiden, den Nitrilen und den Dinitrilen, in Gegenwart eines Katalysators auf Palladium-Basis und gegebenenfalls eines Liganden arbeitet.

**2.** Verfahren gemäß Anspruch 1, dadurch gekennzeichnet, daß das Lösungsmittel unter Dimethylformamid, Acetonitril, Benzonitril, Methylglutaronitril, Adiponitril und 1,6-Dicyanohexan ausgewählt wird.

**3.** Verfahren gemäß Anspruch 1, dadurch gekennzeichnet, daß der Katalysator unter $[(Pd(OAc)_2]$ und

$[Pd(dba)_2]$ ausgewählt wird.

4. Verfahren gemäß Anspruch 1, dadurch gekennzeichnet, daß der Ligand unter Triphenylphosphin und Diphenylphosphinoethan ausgewählt wird.

5. Verfahren gemäß Anspruch 1, dadurch gekennzeichnet, daß man bei einer Temperatur zwischen 0°C und der Rückflußtemperatur des Reaktionsgemisches arbeitet.

6. Die Produkte der Formel

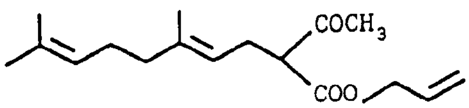
und
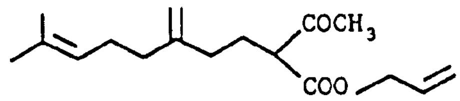

## Claims

1. Process for the preparation of products of formula:

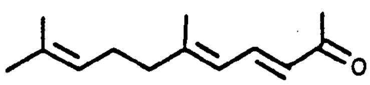
or
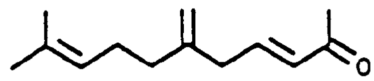

taken alone or as a mixture, characterised in that the oxidative decarboxylation of an allylic β-ketoester of formula:

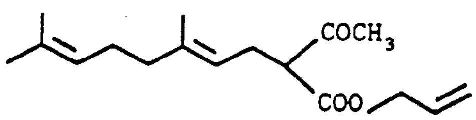
or
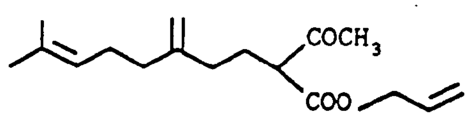

taken alone or as a mixture, is carried out in an organic solvent chosen from the amides, the nitriles and the dinitriles in the presence of a catalyst based on palladium and optionally a ligand.

2. Process according to Claim 1, characterised in that the solvent is chosen from dimethylformamide, acetonitrile, benzonitrile, methylglutaronitrile, adiponitrile and 1,6-dicyanohexane.

3. Process according to Claim 1, characterised in that the catalyst is chosen from [Pd(OAc)z) and $[Pd(dba)_2]$.

4. Process according to Claim 1, characterised in that the ligand is chosen from triphenylphosphine and diphenylphosphinoethane.

5. Process according to Claim 1, characterised in that the reaction is carried out at a temperature between 0°C and the reflux temperature of the reaction mixture.

6. The products of formula:

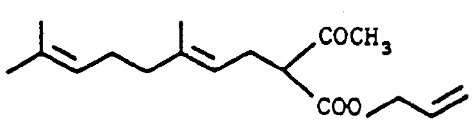
and